(19) Europäisches Patentamt
European Patent Office
Office européen des brevets

(11) Publication number: **0 306 309 B1**

(12) # EUROPEAN PATENT SPECIFICATION

(45) Date of publication of patent specification: **01.12.93**

(51) Int. Cl.$^5$: **C12P 21/08**, C07K 15/00, C12N 5/20, G01N 33/577

(21) Application number: **88308100.2**

(22) Date of filing: **01.09.88**

(54) **Monoclonal antibodies recognizing alpha-hANP and corresponding hybridomas, their preparation and use.**

(30) Priority: **01.09.87 JP 218662/87**

(43) Date of publication of application:
**08.03.89 Bulletin 89/10**

(45) Publication of the grant of the patent:
**01.12.93 Bulletin 93/48**

(84) Designated Contracting States:
**AT BE CH DE ES FR GB GR IT LI LU NL SE**

(56) References cited:
**EP-A- 0 195 331**

**BIOLOGICAL ABSTRACTS, vol. 82, no. 4, 1986, abstract no. 33080, Biological Abstracts Inc., Philadelphia, PA, US; A. JOHN et al.: "The use of a monoclonalantibody to measure plasma atriopeptins in rat",**

**BIOLOGICAL ABSTRACTS/RRM, vol. 35, 1988, abstract no. 103142, Biological Abstracts Inc., Philadelphia, PA, US; M. MUKOYAMA et al.: "Preparation andcharacterization of a monoclonal antibody to alpha-human atrial natriureticpolypeptide"**

(73) Proprietor: **SHIONOGI SEIYAKU KABUSHIKI KAISHA trading under the name of SHIONOGI & CO. LTD.**
**12, 3-chome, Dosho-machi**
**Higashi-ku**
**Osaka(JP)**

(72) Inventor: **Imura, Hiroo**
**59-2, Kitadaimaru-cho**
**Ichijoji Sakyo-ku Kyoto-shi Kyoto(JP)**
Inventor: **Nakao, Kazuwa**
**2-24-6, Oedakitafukunishi-cho**
**Nishikyo-ku**
**Kyoto-shi Kyoto(JP)**
Inventor: **Ishikawa, Eiji**
**3-24-1, Otsukadainishi**
**Miyazaki-shi Miyazaki(JP)**
Inventor: **Kono, Masao**
**3-25-28, Ayukawa**
**Ibaraki-shi Osaka(JP)**
Inventor: **Inouye, Ken**
**1296, Zenkai**
**Ikawadani-cho**
**Nishi-ku**
**Kobe-shi Hyogo(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

American Society of Hypertension Symposium Series (Brenner, B.M. and Laragh, J.H., eds.), vol. 2; Advances in Atrial Peptide Research; Second World Congress, New York, USA, May 1987; Raven Press (publ. 1988), vol. 0, no. 0, pages 191-195

(74) Representative: **Allard, Susan Joyce et al**
**BOULT, WADE & TENNANT**
**27 Furnival Street**
**London EC4A 1PO (GB)**

## Description

This invention relates to kits and a method for the immunoassay of human atrial natriuretic polypeptide (hANP) involving monoclonal antibodies which recognize α-hANP and to hybridomas which produce such monoclonal antibodies for use in the kits and method. More particularly, it relates to kits and a method for the immunoassay of human atrial natriuretic polypeptide involving monoclonal antibodies (and corresponding hybridomas) which recognize the N-terminal half of the ring structure of a α-hANP.

Atrial natriuretic polypeptide (ANP) is present in granules produced by cells of the atrium cordis muscle, and has strong diuretic and natriuretic actions. This sort of polypeptide is found not only in humans but in rats as well, and is called hANP and rANP respectively. Each of hANP and rANP is further classified into 3 types-α, β and γ. α-hANP consists of 28 amino acid residues. Its Cys [7] and Cys [23] that are respectively situated at the 7th and 23rd positions from the N-terminal and form a disulfide linkage, and the sequence between them forms a ring structure (Biochem. Biophys. Res. Commun. (hereafter abbreviated to as BBRC) 118, 131-139, 1984). α-rANP is different from α-hANP only in one residue at the 12th position from N-terminal, which is Ile and Met in α-rANP and α-hANP, respectively (BBRC 117, 839-865, 1983). β-hANP is an antiparallel dimer of α-hANP (Japanese Patent Unexamined Publication No. 184098/1985). γ-hANP consists of 126 amino acid residues, with 99-126 amino acids at the C-terminal corresponding to α-hANP. As regards methods for measuring ANP, radioimmunoassay methods using antiserum have already been established (Science 228, 323-325, 1985; Nature 314, 264-266, 1985; BBRC 124, 815-821, 1984; BBRC 124, 663-668, 1984; BBRC 125, 315-323, 1984). It is known that antiserum CR-3 recognizes the C-terminal fragment [17-28] of ANP.

With respect to a monoclonal antibody recognizing α-hANP, 11A-A11 is known. The said antibody is obtained by using atriopeptin II, a kind of rat ANP, as an antigen, and its epitope is situated between Cys [7] and Ser [25] and involves the disulfide linkage. Thus, the epitope is considered to be a part of the ring structure of ANP. However the affinity of this antibody is not influenced by the difference between Met [12, human] and Ile [12, rat], and the antibody recognizes rANP and hANP (Life Science 38, 1991-1997, 1986).

In the conventional immunoassay of ANP, it has been necessary to extract ANP from plasma samples. Thus, there has been an eager demand for a monoclonal antibody with such a high affinity as to make it possible to achieve a highly sensitive assay without extraction. As a monoclonal antibody recognizing ANP, there is the aforementioned 11A-A11 which recognizes not only hANP but also rANP. Since the antiserum CR-3 for ANP recognizes the C-terminal side of ANP, if an antibody could be provided which specifically recognizes the N-terminal side of ANP, it would be possible to perform sandwich immunoassays using these two kinds of antibody. There is therefore a strong demand for a monoclonal antibody, with a high affinity, which specifically recognizes the N-terminal side of hANP.

Known immunoassays for hANP are radio-immunoassays using radioisotopes (hereafter abbreviated as RIA) and enzyme immunoassays using enzymes (EIA).

American Society of Hypertension Symposium Series, Vol. 2; Advances in Atrial Peptide Research; Second World Congress, New York, USA, May 1987; Raven Press (publ. 1988), Vol 0, no. O. pp191-195 (ANP Sunthesis, storage, and radioimmunoassays (Mukoyama et al) discloses the preparation and characterisation of a monoclonal antibody, KY-ANP-1, to α-ANP. The monoclonal antibody is used in radioimmunoassays (RIAs) to detect α-hANP.

In the case of RIA, there has been a tendency to avoid its use because of the problems of equipment and facilities needed. Thus, there has been an eager demand for the development of a highly sensitive EIA that can be used at any place. In enzyme-labeling, glutaraldehyde has hitherto generally been used as a bridging agent. However, nowadays it is seldom used because of the problems of polymerization and poor yield. As bridging agents to take the place of glutaraldehyde, such agents as the N-hydroxysuccinimidoester of N,N'-O-phenylenedimaleimide and N-(4-carboxycyclohexylmethyl)maleimide [J. Immunoassay 4, 209-327 (1983)] are recent. However, there is no report that these can be applied in an assay of hANP. Thus, in summary, before this invention a highly sensitive immunoassay for hANP did not exist.

Accordingly, the present invention provides a kit for the immunoassay of α-hANP comprising a monoclonal antibody which recognizes the N-terminal portion of the ring structure of α-hANP and an antibody which recognizes the C-terminal side of α-hANP, one of said antibodies being immobilized on a solid phase, and the other being labelled.

The monoclonal antibody of the kit may recognize at least a portion of the sequence α-hANP [7-16] which includes Met [12]. An example of such a monoclonal antibody is one obtainable from a hybridoma obtainable from ECACC 87082001.

The antibody of the kit which recognizes the C-terminal side of α-hANP is obtainable from anti-α-hANP [17-28] antiserum.

The solid phase of the kit may comprise glass beads or polystyrene balls. The label of the antibody may comprise an enzyme.

The present invention also provides a method for the immunoassay of α-hANP in a test sample which comprises mixing with the test sample a monoclonal antibody which recognizes the N-terminal portion of the ring structure of α-hANP and an antibody which recognizes the C-terminal side of α-hANP, one of said antibodies being immobilized on a solid phase, and the other being labelled, allowing the antibodies to react with the α-hANP in the test sample, removing unreacted antibodies from the reaction product and measuring by means of the label, the amount of reacted labelled antibody.

In the measurement of α-hANP by conventional RIA methods, it has hitherto been necessary to extract αhANP from a plasma sample. However, using this invention it is possible directly to measure α-hANP in a test sample without extracting the α-hANP.

Furthermore, such measurements can be performed not only by a two-step method in which an antibody immobilized on a solid phase is first reacted with α-hANP and then a labeled antibody is further reacted; but it can also be performed by a one-step method in which α-hANP and a labeled antibody are reacted at the same time with an antibody immobilized on a solid phase. Thus, α-hANP can be measured easily and it thus becomes possible easily and accurately to diagnose and follow up heart disease, kidney diseases, hypertension (essential and secondary), edematous diseases (cirrhosis of the liver, nephrosis, catapleptic edema, etc.), and dehydration accompanied by abnormalities in the balance of body fluids.

In the accompanying drawings:-

Figure 1 shows cross reactivities of α-hANP, α-rANP and α-hANP fragments with a monoclonal antibody for use in a kit or method of this invention. Figure 2 illustrates a standard curve (○) of α-hANP and dilution curves (●, ▲, ■) of plasma, by EIA. Fig. 3 shows a standard curve of α-hANP (○) and dilution curves (●, ▲, ■) of plasma, by a one-step EIA.

(1) Preparation of a hybridoma producing a monoclonal antibody

α-hANP is a polypeptide consisting of 28 amino acid residues, and its molecular weight is comparatively low. Consequently, its ability to induce antibody-production (immunogenicity) is low. For this reason, in order to use it as an antigen, it is combined with bovine serum albumin or bovine thyroglobulin. The conjugate obtained is emulsified with a suitable adjuvant such as Freund's complete adjuvant, and is then used for immunizing mice.

Immunization is performed by inoculating the above emulsion into mice several times at intervals of a few weeks intraperitoneally, subcutaneously or intravenously. Three to five days after the last immunization, the spleen is taken out, which is used as a source of antibody-producing cells. At the same time, as the other parent cells to be fused with the antibody-producing cells to make hybridomas, a myeloma cell strain having a suitable marker such as hypoxanthine-guaninephosphoribosyl transferase-deficiency (HGPRT-) or thymidine kinase-deficiency (TK-) is prepared, and the myeloma cell strain is then fused with the antibody-producing cells to make a hybridoma.

As the culture medium for making the hybridoma, there are various examples such as Eagle's MEM, Dulbecco's modified medium, and RPMI-1640, which are generally used with the addition of about 15% fetal calf serum (FCS) as occasion demands.

First, myeloma, as parent cells, and spleen cells are prepared at the ratio of 1:6.5. As for a fusing agent, 50% polyethylene glycol (PEG) is generally used because of the high fusing rate. Fused strains are selected by the HAT selection method. The producing hybridomas are screened according to such known methods as the membrane fluorescence antibody technique, the enzyme linked immunosorbent assay method (ELISA method) or the immune tissue staining method by using the culture supernatant. Thereby, clones of hybridomas producing an objective immunoglobulin are selected. To make the hybridomas monoclonal, normal spleen cells are placed as a feeder layer in a 96-wel microwell at $10^5$ cells/well, where hybridomas are placed less than one cell/well, and on clones which grow the screening is again performed. Monoclonal hybridomas are obtained by repeating such sub-cloning.

(2) Production of monoclonal antibody

To make a monoclonal antibody for use in a kit or method of this invention, a hybridoma obtained above is cultured in vitro or in vivo. When culturing in vitro, usual media such as mentioned above may be used with the addition of FCS. After culturing in the medium for 3 to 5 days, the monoclonal antibody is obtained from the culture supernatant. In case of in vivo culture, the hybridoma is inoculated into the abdominal cavity of a mammal. On day 7 to 14 after that, ascites are collected, from which the monoclonal

antibody is obtained. As compared with in vitro culture, in vivo culture produces a far larger quantity of antibody effectively. Thus, in vivo culture is preferred.

The monoclonal antibody thus-obtained from a culture supernatant or ascites is purified by a suitable combination of known methods such as ammonium sulfate fractionation, use of a DEAE Sepharose column, etc. Thus for instance, the purification may be performed in a manner such as described in the following Example.

Thus, a general method for producing an antibody for use in a kit or method of the invention or a hybridoma for producing such an antibody comprises fusing myeloma cells with antibody-producing cells obtained from an animal immunized with an immunogenic preparation of α-hANP and selecting for antibody to α-hANP-producing hybridomas; and, if desired, culturing a resulting hybridoma to produce monoclonal antibody.

As will be shown later in the Example, the preferred monoclonal antibody KY-ANP-1 obtained for use in this invention specifically recognizes α-hANP, and it shows almost no affinity with rANP. It is presumed that its epitope is in the N-terminal half of the ring structure of α-hANP, more particularly, a part covering from Cys [7] to Gly [16]. Further, since it reacts very weakly with rANP, it epitope is considered to be a part containing Met [12]. Since the said epitope is a part common not only to α-hANP but also to β-hANP and γ-hANP, the said antibody also shows reactivity with β-hANP and γ-hANP.

Furthermore, this antibody shows a high affinity (Ka = $3.1 \times 10^{10}$ $M^{-1}$) with α-hANP. As shown in the standard curve of α-hANP in Fig. 1, 10% and 50% inhibitory concentrations ($IC_{10}$ and $IC_{50}$) were 10 pg/tube and 100 pg/tube, respectively.

The hybridoma KY-ANP-I which produces the monoclonal antibody KY-ANP-I for use in this invention has been deposited with PHLS Centre for Applied Microbiology & Research, European Collection of Animal Cell Cultures (ECACC), Porton Down, Salisbury, SP4 OJG, England, since Aug. 20, 1987 under an accession No. 87082001 in accordance with the Budapest Treaty.

(3) Conjugation of antibody to carrier

As a solid phase for conjugating an antibody to a carrier, there may be used such items as glass or plastic beads, balls, tubes, or plates, which are commercially available and are generally used in immunoassays as carriers for antigen/antibody reactions. An antibody recognizing the N-terminal side or C-terminal side of α-hANP may be adsorbed to any of these carriers. The adsorption is usually performed overnight in phosphate buffer at pH 6 - 10, preferably around neutral pH, at room temperature. The carrier on which the antibody been adsorbed is kept with cooling in the presence of an antiseptic, such as sodium azide.

Both monoclonal antibodies and polyclonal antibodies can be adsorbed on carriers in the same manner as above.

(4) Rabbit anti α-hANP [17 - 28] serum

Rabbits are immunized several times with α-hANP [17 - 28]-bovine thyroglobulin conjugate prepared by the carbodiimide method, and 10 to 14 days after the last immunization, the blood is collected, from which rabbit anti-α-hANP [17 - 28] serum is prepared.

(5) Prepared of enzyme-labeled antibody

Rabbit IgG, $F(ab')_2$ and Fab′

Antiserum prepared as above is fractionated with sodium sulfate, which is then passed through DEAE-cellulose column, whereby IgG is obtained. The IgG obtained is digested with pepsin to make $F(ab')_2$, which is then reduced with 2-mercaptoethylamine to obtain anti-α-hANP [17 - 28] Fab′. Prepared of Fab′ from IgG is detailed in J. Immunoassay 4, 209 -327 (1983), and the same procedures can be used in this invention.

Enzyme-labeling of antibodies

As an enzyme to label an antibody, there may be used alkaline phosphatase, β-D-galactosidase, peroxidase, glucose oxidase, etc. In this invention, horseradish peroxidase is preferably used. As a bridging agent, there may be used N,N′-o-phenylenedimaleimide, N-succinimidyl 4-(N-malemidomethyl)-cyclohexanoate, N-succinimidyl 6-maleimidohexanoate, N-succinimidyl 3-(2-pyridyldithio)propionate, 4, 4′-dithiodipyridine, and others already known.

The reactions of these bridging agents with enzymes and antibodies may be performed by known methods depending on the characteristics of each agent. Depending on circumstances, antibody fragments such as Fab′, Fab, and $F(ab')_2$ are used as antibody. As bridging agent in this invention, it is desirable to use N-succinimidyl 4-(N-maleimidomethyl)cyclohexanoate, or N-succinimidyl 6-maleimidohexanoate. Furthermore, regardless of whether the antibodies are polyclonal or monoclonal, enzyme-labeled antibodies can be obtained by the same procedures. Therefore, enzyme-labeled antibodies obtained by using the aforementioned two bridging agents may be represented by the following general formula (I):

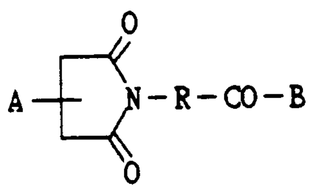

(I)

[wherein A shows an antibody or its fragment e.g. which recognizes the N-terminal side or C-terminal side of α-hANP, B indicates a labeling enzyme, and R stands for 4-methylenecyclohexyl or pentamethylene]

It is desirable that the enzyme-labeled antibodies thus obtained are purified by affinity chromatography so that a more highly sensitive immunoassay system is attained. The purified enzyme-labeled antibodies are stored in a cool and dark place with a stabilizer such as thimerosal or glycerol, or after being lyophilized.

When α-hANP is measured by using an immunoassay reagent prepared as above, a combination of antibodies may be arranged as follows. In cases where an antibody recognizing the N-terminal side of α-hAHP is immobilized, an antibody recognizing the C-terminal side is enzyme-labeled.

In immobilizing an antibody, generally a comparatively large quantity of antibody is required. Therefore, for the immobilization a monoclonal antibody which can steadily be obtained in a large quantity (for instance, KY-ANP-I) is suitable.

However, a polyclonal antibody prepared from antiserum can also be used without inconvenience.

As regards the antibody to be enzyme-labelled, either a monoclonal antibody or a polyclonal antibody may be used, provided it is one recognizing a site different from that recognized by the immobilized antibody. For example, when using KY-ANP-I as an immobilized antibody, the above-mentioned antiserum CR-3 or antiserum F36 mentioned in the Example can be applied as an enzyme-labeled antibody.

The reverse of this combination can also be applied. Of course, monoclonal antibodies recognizing the C-terminal side of α-hANP and antisera recognizing the N-terminal side of αhANP can also be applied in this invention.

In general, the invention provides the use of an antibody in a kit or method of the invention for the immunoassay of α-hAHP, preferably in in vitro diagnosis.

EXAMPLE

Preparation of hybridoma

Synthetic α-hANP (1.5 mg) and bovine thyroglobulin (5.4 mg) were dissolved in 2 ml of distilled water. To this solution was added dropwise a solution of 30 mg of 1-ethyl-3-(3-dimethylaminopropyl)-carbodiimide in 1 ml of distilled water over a period of 10 min at room temperature, after which the mixture was stirred at room temperature for 24 hr. This solution was dialyzed 6 times against 3 ℓ of distilled water over a period of 3 days. The dialyzate was divided into 5 portions, which were stored at -20 °C (See BBRC 124, 815 - 821, 1984).

To the above solution stored in divided portions (containing 300 μg of α-hANP) was added distilled water to make 1.2 ml, which was then suspended in 1.2 ml of Freund's complete adjuvant. Of this, about 2 ml was injected intraperitoneally and subcutaneously into 10 BALB/c female mice (200 μl per animal). The animals were given additional immunization in the same manner 3 weeks later. Five weeks after the additional immunization, the antibodies in the blood were measured, and to two mice which showed the most prominent immune reaction was given further additional immunization through the tail vein with above solution containing 50 μg of α-hANP. Four days after that, the spleen cells were collected from the two

mice for cell fusion.

The spleen cells collected (1.3 x $10^8$ pieces) and myeloma cells X63-Ag8.653 (2 x $10^7$ pieces) were mixed in Dulbecco's medium (DMEM), and the mixture was centrifuged at 1500 rpm for 5 min at 4°C. The pellets obtained were melted by warming at 37°C, and then 1 ml of 50% PEG 4000 (PEG 1 g/DMEM 1 ml) was added dropwise over a period of 1 min. Then, the mixture was allowed to stand for 2 min at 37°C, after which the solution was diluted by adding dropwise 10 ml of DMEM of 37°C over a period of 5 min. This was then washed by centrifugation at 4°C with DMEM containing 15% FCS.

The cells obtained were sown on a 96-well plate, and cultured for 2 weeks in HAT medium, and further for 1 week in HT medium. Of a total of 768 wells, hybridomas proliferated in about 30% (212/768), 4% (8/212) of which was found to produce α-hANP antibodies.

Cells in the wells showing the highest antibody titer were cloned by limiting dilution method. Namely, as feeder cells, BALB/c mouse thymus cells were added to the wells at $10^5$ pieces/well and hybridomas were placed therein at 1 piece/well and cultured there. This procedure was performed twice.

By such cloning, a clone producing the largest quantity of antibody steadily was selected, and it was named KY-ANP-I.

The antibody titers in the antiserum obtained from the immunized mice and in the supernatant of the hybridoma culture were measured as follows:

Antiserum of immunized mouse of supernatant of hybridoma culture was taken as sample. A mixture of 100 µl of the diluted solution of the said sample, 300 µl of assay buffer (RIA buffer), 100 µl of $^{125}$I-α-hANP (10000 cpm) was allowed to undergo reaction for 24 hr at 4°C, after which the solution was mixed with 1 ml of dextran-coated-charcoal, and the mixture was allowed to react for 5 min at 4°C. After that, the mixture was centrifuged at 3000 rpm for 30 min at 4°C, and the radioactivity of its supernatant was measured with a γ -counter, from which the antibody titer in the diluted solution of the sample was calculated.

The above-mentioned $^{125}$-I-α-hANP was prepared by Chloramine T method. Namely, α-hANP (1 µg) was mixed with Na$^{125}$I (1 mCi), to which was added 10 µl of chloramine T (5.25 mg/ml), and 10 sec after that, 20 µl of sodium pyrosulfite (4.5 mg/ml) was added. To this was further added 1 ml of 2% gelatin, which was then purified with Sep-Pak C18 (made by Waters).

Preparation of monoclonal antibody

BALB/c mice were pretreated twice at intervals of 1 to 2 weeks by intraperitoneally giving pristane at 0.5 ml per animal at a time. To each of said mice was then intraperitoneally injected 5 x $10^5$ pieces of hybridoma KY-ANP-I suspended in 200 µl of DMEM. Ascites obtained was purified with Protein A-Sepharose® CL-4B column to obtain monoclonal antibody KY-ANP-I.

Properties of KY-ANP-I

The isotype of this monoclonal antibody was determined by Ouchterlony's method to be $IgG_1$. Its affinity was obtained by making a Scatchard plot based on radiobinding assay, and as a result, it was found the Ka = 3.1 x $10^{10}$ $M^{-1}$.

Its epitope was determined by measuring its cross reactivity with various ANP-related peptides by RIA, and the result is shown in Fig. 1. Since it shows almost no reaction with α-ANP [17 - 28] and α-ANP [ 1 - 6], the epitope is presumed to be contained in α-ANP [7 -16]. Besides, it reacts very weakly with α-hANP [8 - 22] or α-rANP. Therefore, it is presumed that it is necessary for the epitope to have a ring structure and that the epitope contains Met. It was concluded that the epitope recognized by the monoclonal antibody of this invention is nearly N-terminal half of the ring structure of α-hANP.

Meanwhile, reactivities of KY-ANP-I with β-hANP and γ-hANP were examined by the above-mentioned method for measuring antibody titer. As a result, it was found that KY-ANP-I recognizes not only α-hANP but also γ-hANP, and further that it reacts with β-hANP with 80% cross reactivity.

Preparation of anti α-hANP [17 - 28] bovine thyroglobulin serum

α-hANP [17 - 28] was bound to thyroglobulin by the known carbodiimide method (BBRC 117, 695, 1984) To 0.5 ml of an aqueous solution containing 3.1 mg of α-hANP [17 - 28] was added 0.7 ml of an aqueous solution containing 19 mg of bovine thyroglobulin. To this was added 1 ml of an aqueous solution containing 40 mg of 1-ethyl-3-(3-dimethylaminopropyl)carbodiimide hydrochloride, and the mixture was stirred for 2 hr under cooling with ice. The reaction solution was dialyzed 3 times at 4°C against 2 ℓ of distilled water, after which it was lyophilized to obtain 21 mg of the conjugate. The bound molar ratio of α-hANP [17 - 28] to bovine thyroglobulin was obtained by amino acid analysis method to be 30.

Immunization and blood collection

In 0.25 ml of physiological saline solution was dissolved 0.25 mg of the above-mentioned α-hANP [17 - 28]-bovine thyroglobulin conjugate. This was suspended in an equal quantity of Freund's complete adjuvant, and the solution of subcutaneously injected into a rabbit at 20 or more sites on the back. This procedure was performed 6 times at intervals of 3 weeks, and on the 10th day after the last injection, the rabbit was exsanguinated from the carotid artery to obtain antiserum F36.

Preparation of rabbit IgG

To 1 ml of anti α-hANP serum (F36) was slowly added 0.18 g of sodium sulfate with stirring, and after the added material was completely dissolved, stirring was continued at 22 - 25 °C. The mixture was then centrifuged at 10,000 rpm for 10 min at 22 - 25 °C. The precipitate was dissolved in 1 ml of sodium phosphate buffer (pH 6.3; 17.5 mmol/ℓ). which was then dialyzed against the same buffer. The supernatant was passed through DEAE cellulose column which had been equilibrated beforehand with the same buffer (pH 6.3; 17.5 mmol/ℓ). The wet volume of the DEAE cellulose required for passing 10 mg of protein in the supernatant is 1 ml. The quantity of IgG obtained was 7 mg, when calculated by using the extinction coefficient at 280 nm 1.5 $g^{-1}$ ℓ $cm^{-1}$ and the molecular weight of IgG 150,000 (See J. Immunoassay 4, 209 - 327 (1983)).

Preparation of $F(ab')_2$

Ten mg of rabbit IgG was dialyzed at 5 °C against 1 ml of sodium acetate buffer (pH 4.5, 0.1 mol/ℓ). To the dialyzed IgG solution was added 0.05 ml (1/20 volume) of an aqueous solution of sodium chloride (2 mol/ℓ). In this was dissolved pepsin (0.2 mg/10mg IgG) derived from the mucous membrane of swine stomach. The mixture was allowed to react for 15 - 24 hr at 37 °C. Then, the mixture was adjusted to pH 8 with an aqueous solution of sodium hydroxide (1 mol/ℓ), after which the mixture was applied to a column of Sephadex® G-150 (1.5 x 45 cm for 1.0 - 1.5 ml, 2.0 x 45 cm for 2.0 - 2.5 ml) and eluted with sodium borate buffer (pH 8.0, 0.1 mol/ℓ). The quantity of $F(ab')_2$ obtained was 6 mg, when calculated by using 1.48 $g^{-1}$ ℓ $cm^{-1}$ as the extinction coefficient at 280 nm and 92,000 as molecular weight of $F(ab')_2$ (See J. Immunoassay, 4, 209 -327 (1983)).

Preparation of Fab′

Three mg of $F(ab')_2$ was dissolved in 0.45 ml of sodium phosphate buffer (pH 6.0, 0.1 mol/ℓ). To this was added 2-mercaptoethylamine/sodium phosphate buffer solution (pH 6.0, 0.1 mol/ℓ) containing 5 mmol/ℓ of EDTA the buffer solution being prepared immediately before use. Then, the mixture was allowed to react for 1.5 hr at 37 °C. After that, the reaction mixture was applied to a column of Sephadex G -25 (1 x 30 cm) and eluted with sodium phosphate buffer (pH 6.0, 0.1 mol/ℓ; containing 5 mmol/ℓ of EDTA). The quantity of Fab′ obtained was 2.5 mg, when calculated by using 1.48 $g^{-1}$ ℓ $cm^{-1}$ as the extinction coefficient at 280 nm and 46,000 as the molecular weight of $F(ab')_2$ (see J. Immunoassay, 4, 209 - 327 (1983)).

Preparation of peroxidase-labeled anti-α-hANP [17 - 28] Fab′

Two mg of horseradish peroxidase (50 nmol) was dissolved in 0.3 ml of sodium phosphate buffer (pH 7.0, 0.1 mol/ℓ). To this was added a mixture of 0.65 mg (2100 nmol) of N-succinimidyl 6-maleimidohexanoate and 0.03 ml of N,N-dimethylformamide, and the mixture was allowed to react with stirring for 0.5 - 1 hr at 30 °C. After that, the reaction mixture was subjected to centrifugation so that excess of reagent was removed as precipitate. The supernatant was passed through a Sephadex® G-25 column (1.0 x 45 cm), and was eluted with sodium phosphate buffer (pH 6.0, 0.1 mol/ℓ) at a flow rate of 30 - 40 ml/h wherein the volume of each fraction was set 0.5 - 1.0 ml. A column of Sephadex® G-50 (fine, Pharmacia) (1.0 x 6.4 cm, 5 ml) with a fine mesh filter at the bottom was equilibrated with the above buffer and centrifuged in a test tube at 100 x g for 2 min. The above reaction product (0.5 ml) was applied to this column and was centrifuged in the same manner. The fraction obtained was concentrated by centrifuging it with microconcentrator (CENTRICON-30®, Amicon Corp.) at 2,000 x g at 4 °C.

The sodium phosphate buffer (pH 6.0, 0.1 mol/ℓ) was dissolved 1.8 mg (45 nmol) of maleimide peroxidase conjugate thus prepared. To this was added a solution of about 2.0 mg (43 nmol) of Fab′ in 0.2 - 0.4 ml of sodium phosphate buffer (pH 6.0, 0.1 mol/ℓ) containing 5 mmol/ℓ EDTA and the mixture was allowed to react at 4°C for 20 hr or at 30°C for 1 hr. The final concentration of the maleimide peroxidase Fab′ conjugate in the reaction mixture was set at 50 -100 μmol/ℓ. This reaction mixture was passed through a column (1.5 x 45 cm) of Ultrogel® AcA 44 and eluted with sodium phosphate buffer (pH 6.5, 0.1 mol/ℓ) at a flow rate of 0.3 - 0.5 ml/min, with the volume of each fraction set at about 1.0 ml. In this way, about 2.5 mg of objective peroxidase-labeled anti-α-hANP [17 - 28] Fab′ was obtained. (See J. Immunoassay, 4, 209 - 327 (1983)).

α-hANP [17 - 28]-nonspecific rabbit IgG conjugate

A solution of α-hANP [17 - 28] (0.5 mg) in 0.2 ml of sodium phosphate buffer (0.1 mol/ℓ, pH 7.0) was allowed to react with 0.01 ml of 105 mmol/ℓ N-succinimidyl 6-maleimidohexanoate in N,N′-dimethylformamide at 30°C for 30 min. The reaction products was subjected to gel filtration with a column of Sephadex® G-10 (1.0 x 45 cm) using 0.1 mol/ℓ sodium phosphate buffer (pH 6.0) containing 5 mmol/ℓ) EDTA. The average value of the maleimide groups introduced into α-hANP [17 - 28] was 0.6/molecule.

A solution of nonspecific rabbit IgG (10 mg) in 0.1 mol/ℓ sodium phosphate buffer (0.6 ml, pH 7.5) was allowed to react with a solution of 210 mmol/ℓ S-acetylmercaptosuccinic anhydride (0.03 ml) in N,N′-dimethylformamide for 30 min at 30°C. To this reaction mixture were added 0.1 mol/ℓ tris hydrochloride buffer (0.1 ml, pH 7.0), 0.1 mol/ℓ EDTA (0.02 ml) and 1 mol/ℓ hydoxylamine hydrochloride (0.1 ml, pH 7.0), and the mixture was allowed to react for 5 min at 30°C. The reaction product was subjected to gel filtration with a column of Sephadex® G-25 (1.0 x 45 cm) using 0.1 mol/ℓ sodium phosphate buffer (pH 6.0) containing 5 mmol/ℓ EDTA. The average value of the thiol groups introduced into nonspecific rabbit IgG was 11/molecule.

An aliquot (2.0 ml) of the above maleimide-α-hANP [17 - 28] was allowed to react at 30°C for 30 min with mercaptosuccinylated nonspecific rabbit IgG (2.4 mg) obtained in 0.1 mol/ℓ sodium phosphate buffer (pH 6.0, 0.25 ml) containing 5 mmol/ℓ EDTA. To the reaction product 0.1 ml of N-ethylmaleimide 0.01 mmol/ℓ was added so as to block the remaining mercapts groups. The mixture was then subjected to gel filtration with a column of Sephadex® G-25 (1.0 x 45 cm) using 0.1 mol/ℓ sodium phosphate buffer (pH 7.0). The average number of the molecules of the α-hANP [17 - 28] which combined with nonspecific rabbit IgG was 8.7/molecule, calculated from the reduction of the thiol groups.

Purification of horseradish peroxidase-labeled anti-hANP [17 - 28] Fab′

α-hANP [17 - 28]-nonspecific rabbit IgG conjugate (2 mg), bovine thyroglobulin (10 mg), and mouse serum protein (20 mg) each was combined with cyanogen bromide-activated Sepharose® 4B (1g). Horseradish peorxidase-labeled anti-hANP [17 - 28] Fab′ (7.8 mg) in 0.8 ml of 0.1 mol/ℓ sodium phosphate buffer (pH 6.5) containing 1 g/ℓ gelatin and 50 mg/ℓ thimerosal was passed through two columns, one is a column of bovine thyroglobulin-Sepharose® 4B (0.55 x 4.0 cm) and the other is a column of mouse serum protein-Sepharose® 4B (0.55 x 4.0 cm), at a flow rate of 0.5 ml/h, using 10 mmol/ℓ sodium phosphate buffer (pH 7.5) containing 1g/ℓ gelatin and 0.1 mol/ℓ sodium chloride. Then, purification was further made with a column of α-hANP [17 - 28]-nonspecific rabbit IgG-Sepharose® 4B (0.35 x 2.0 cm) using 3.2 mmol/ℓ hydrochloric acid (pH 2.5). The eluate (1 ml) containing 0.35 mg of purified labeled substance was immediately mixed with 0.1 ml of 1 mol/ℓ sodium phosphate buffer (pH 7.0) and 0.01 ml of 100 g/ℓ gelatin. The quantity of the labeled substance was about 0.35 mg, calculated from the peroxidase activity.

Preparation of monoclonal anti-α-hANP-coated polystyrene balls

Fifty pieces of polystyrene ball (diameter 3.2 mm, made by Precision Plastic Ball Co., Chicago U.S.A.) were put into 1 ml of 0.1 M sodium phosphate buffer (pH 7.0), to which was added 100 μg/ml of monoclonal anti-α-hANP-$IgG_1$ (KY-ANP-I), and the mixture was left overnight at room temperature. Then, the polystyrene balls were washed with sodium phosphate buffer (pH 7.0), after which sodium azide was added by 0.1%. The polystyrene balls thus prepared were preserved in a refrigerator.

Collection of Plasma

Blood was collected at 9:00 am from the antecubital vein of healthy male subjects (aged 26 - 32) in a supine position after overnight fast. Blood was collected again from the same subjects in the same manner after one-hour walking with intravenous administration of 40 mg of furosemide. The blood was taken with chilled plastic syringes and was transferred to chilled disposable siliconized glass tubes changed with aprotinin and EDTA. It was then centrifuged (500 x g) to spearate plasma. The final concentrations of aprotinin and EDTA were 1,000 kallikrein inactivator units (KIU)/ml and 1 mg/ml respectively.

Enzyme immunoassay of α-hANP by sandwich method

One piece of monoclonal anti α-hANP $IgG_1$ (KY-ANP-I)-coated polystyrene ball was put into standard solution of α-hANP or into plasma (total volume 0.15 ml), which was allowed to stand for 24 hr at 4°C. The α-hANP standard solution was diluted with 10 mM sodium phosphate buffer (pH 7.0; containing 1 mg/ml gelatin, 0.3 M sodium chloride, 0.2 mM cystine, 1 mM EDTA, 1 mg/ml sodium azide, and 1,000 KIU/ml aprotinin) to a final volume of 0.15 ml. The plasma (50 µl) was mixed with 0.1 ml of 10 mM sodium phosphate buffer (pH 7.0; containing 1 mg/ml gelatin, 0.4 M sodium chloride, 0.3 mM cystine, 1.5 mM EDTA, 1.5 mg/ml sodium azide, and 1,000 KIU/ml aprotinin).

After removing the liquid portion from the reaction product, the polystyrene ball was washed twice with 2 ml of 10 mM sodium phosphate buffer (pH 7.0; containing 0.1 M sodium chloride). Then, the ball was mixed with 50 ng of rabbit anti-α-hANP [17 - 28] Fab′-peroxidase conjugate purified with affinity chromatography and 0.15 ml of 10 mM sodium phosphate buffer (pH 8.0; containing 1 mg/ml gelatin, 0.2 mM cystine and 1 mM EDTA), and the mixture was allowed to stand for 24 hr at 4°C. After removing the liquid portion, the polystyrene ball was washed twice in the same manner as mentioned above and was transferred to another test tube. In order to determine the activity of the conjugated peroxidase, the fluorescence intensity was measured after allowing it to react at 30°C for 60 min with 3-(4-hydroxyphenyl)-propionic acid used as substrate. The fluorescence intensity was measured based upon 200 ng/ml quinine/50 mM sulfuric acid as base.

Specificity

In EIA this method, the standard dilution curve of α-hANP was identical with those of α-hANP [4 - 28], α-hANP [5 - 28] and α-hANP [7 - 28], which indicated no effect of N-terminal modifications. On the other hand, deletion of C-terminal amino acids considerably reduced the reactivity. However, no reaction was observed with terminal fragments: α-hANP [17 - 28] and α-hANP [1 - 6]. The cross reactions with β-hANP and α-rANP were 4.7% and 0.01% respectively on the molar basis. These results were consistent with the specificity of the antibodies used: mouse monoclonal $IgG_1$ immobilized on polystyrene ball was specific to the N-terminal half of the ring structure of α-hANP, while rabbit Fab′ conjugated to peroxidase was specific to α-hANP [17 - 28].

Influence of plasma

The bound oxidase activity (nonspecific binding) measured in the absence of plasma and α-hANP was identical with peroxidase activity measured in the presence of plasma pretreated with 1 pmol of monoclonal anti-α-hANP IgG, (KY-ANP-I). The recoveries of α-hANP (10 - 200 pg/ml) added to plasma containing 4.3 - 332 pg/ml ANP were 81 - 94%. The dilution curve of plasma was parallel with the standard curve of α-hANP obtained in the absence of plasma, when the plasma volume per tube was within the range of 1 - 50 µl. Thus, little plasma interference was observed when the volume of plasma used was 50 µl or less. The result is shown in Fig. 2.

Sensitivity of EIA

The detection limit of α-hANP was 30 fg (10 amol)/tube. When 50 µl of plasma was used, the minimal detection level of plasma α-hANP was 0.6 pg/ml (0.2 fmol/ml). This sensitivity of EIA was much higher, one to two orders of magnitude times, than those of conventional RIA.

Reproducibility of EIA

The reproducibility of this invention was defined at 5 different levels over the range of 5 - 158 pg/ml of α-hANP in plasma. Coefficients of variations in reproducibility for within-assay and between-assay were 3.2 - 9.4% (n = 20) and 5.4 - 12.0% respectively.

Plasma α-hANP levels in healthy subjects in the basal and blood volume-contracted states by EIA, as compared with those by RIA

The basal plasma α-hANP level determined by EIA in healthy subjects was 24.5 ± 5.9 pg/ml. The plasma α-hANP level decreased to 15.3 ± 3.7 pg/ml in the blood volume-contracted state after one-hour walking with intravenous administration of furosemide (40 mg). Plasma α-hANP levels determined simultaneously by RIA were 28.2 ± 5.7 pg/ml and 18.3 ± 3.2 pg/ml respectively. The following corelationship was noted between measurements of α-hANP by EIA (y) and RIA (x):

$y - 0.78x + 1.3,$
$r = 0.92, n = 24$

As is clear from the above data, EIA by the method of this invention is so sensitive that even in the blood volume-contracted state it is possible to measure the plasma α-hANP concentration without extraction.

Plasma α-hANP levels in patients with heart diseases

Plasma α-hANP levels measured with the α-hANP measuring reagent of this invention in patients with heart diseases were as shown in the following Table 1:

Table 1

| Patient No. | Level obtained (pg/ml) |
|---|---|
| 1 | 690 |
| 2 | 470 |
| 3 | 332 |
| 4 | 129 |
| 5 | 113 |
| 6 | 108 |
| 7 | 89 |
| 8 | 78 |
| 9 | 52 |
| 10 | 35 |
| Remarks: Patients Nos. 1 - 3 had severe heart failure. | |

Application to one-step method in EIA

The EIA in this invention can be performed by one-step method, too. Fig. 3 shows standard curve of α-hANP and dilution curves of plasma by one-step method. In this method, hANP, enzyme-labeled antibody, and antibody-coated polystyrene balls were mixed all at the same time, and reaction was allowed to take place at 4 ° C for 24 hr. Peroxidase (POD) activity was measured at 30 ° C for 30 min. Other conditions were same as those of the two-step method.

**Claims**

1. A kit for the immunoassay of α-hANP comprising a monoclonal antibody which recognizes the N-terminal portion of the ring structure of α-hANP and an antibody which recognizes the C-terminal side of α-hANP, one of said antibodies being immobilized on a solid phase, and the other being labelled.

**2.** A kit as claimed in claim 1, wherein said monoclonal antibody recognizes at least a portion of the sequence α-hANP [7-16] which includes Met [12].

**3.** A kit as claimed in claim 1 or claim 2, wherein said monoclonal antibody is obtainable from a hybridoma obtainable from ECACC 87082001.

**4.** A kit as claimed in any one of claim 1 to 3, wherein said antibody recognizing the C-terminal side of α-hANP is obtainable from anti-α-hANP [17-28] antiserum.

**5.** A kit as claimed in any one of claims 1 to 4 wherein the solid phase comprises glass beads or polystyrene balls.

**6.** A kit as claimed in any one of claims 1 to 5 wherein the label comprises an enzyme.

**7.** A method for the immunoassay of α-hANP in a test sample which comprises mixing with the test sample a monoclonal antibody which recognizes the N-terminal portion of the ring structure of α-hANP and an antibody which recognizes the C-terminal side of α-hANP, one of said antibodies being immobilized on a solid phase, and the other being labelled, allowing the antibodies to react with the α-hANP in the test sample, removing unreacted antibodies from the reaction product and measuring by means of the label, the amount of reacted labelled antibody.

**Patentansprüche**

**1.** Kit für den Immunassay von α-hANP umfassend einen monoklonalen Antikörper, der den N-terminalen Teil der Ringstruktur von α-hANP erkennt, und einen Antikörper, der die C-terminale Stelle von α-hANP erkennt, wobei einer der Antikörper auf einer Feststoffphase immobilisiert ist und der andere markiert ist.

**2.** Kit nach Anspruch 1, wobei der monoklonale Antikörper mindestens einen Teil der α-hANP [7-16]-Sequenz, welche Met [12] umfasst, erkennt.

**3.** Kit nach Anspruch 1 oder Anspruch 2, wobei der monoklonale Antikörper aus einem aus ECACC 87082001 erhältlichen Hybridom erhältlich ist.

**4.** Kit nach einem der Ansprüche 1 bis 3, wobei der Antikörper, der die C-terminale Stelle von α-hANP erkennt, aus Anti-α-hANP [17-28]-Antiserum erhältlich ist.

**5.** Kit nach einem der Ansprüche 1 bis 4, wobei die Feststoffphase Glaskugeln oder Polystyrolkugeln enthält.

**6.** Kit nach einem der Ansprüche 1 bis 5, wobei die Markierung ein Enzym umfasst.

**7.** Verfahren für den Immunassay von α-hANP in einer Testprobe, welches Mischen eines monoklonalen Antikörpers, der den N-terminalen Teil der Ringstruktur von α-hANP erkennt, und eines Antikörpers, der die C-terminale Stelle von α-hANP erkennt, wobei einer der Antikörper auf einer Feststoffphase immobilisiert ist und der andere markiert ist, mit der Testprobe, Ermöglichen, dass sich die Antikörper mit dem α-hANP in der Testprobe umsetzen, Entfernen der nicht-umgesetzten Antikörper aus dem Reaktionsprodukt und Messen der Menge des umgesetzten markierten Antikörpers mit Hilfe der Markierung, umfasst.

**Revendications**

**1.** Trousse pour doser par analyse immunologique l'α-hANP, comprenant un anticorps monoclonal qui reconnaît la portion N-terminale de la structure cyclique de l'α-hANP et un anticorps qui reconnaît le côté C-terminal de l'α-hANP, l'un de ces anticorps étant immobilisé sur une phase solide, et l'autre étant marqué.

2. Trousse selon la revendication 1, dans laquelle ledit anticorps monoclonal reconnaît au moins une portion de la séquence [7-16] de l'α-hANP, qui comprend Met [12].

3. Trousse selon la revendication 1 ou 2, où l'anticorps monoclonal peut être obtenu à partir d'un hybridome pouvant être obtenu à partir d'ECACC 87082001.

4. Trousse selon l'une quelconque des revendications 1 à 3, dans laquelle l'anticorps reconnaissant le côté C-terminal de l'α-hANP peut être obtenu à partir d'un antisérum anti-α-hANP [17-28].

5. Trousse selon l'une quelconque des revendications 1 à 4, dans laquelle la phase solide comprend des perles de verre ou des billes de polystyrène.

6. Trousse selon l'une quelconque des revendications 1 à 5, dans laquelle le marquage comprend une enzyme.

7. Procédé de dosage de l'α-hANP par analyse immunologique dans un échantillon d'essai, qui consiste à mélanger à l'échantillon d'essai un anticorps monoclonal qui reconnaît la portion N-terminale de la structure cyclique de l'α-hANP et un anticorps qui reconnaît le côté C-terminal de l'α-hANP, l'un de ces anticorps étant immobilisé sur une phase solide, et l'autre étant marqué, à laisser les anticorps réagir avec l'α-hANP dans l'échantillon d'essai, à enlever du produit de la réaction les anticorps intacts et à mesurer au moyen du marquage la quantité d'anticorps marqués ayant réagi.

FIG. 1.

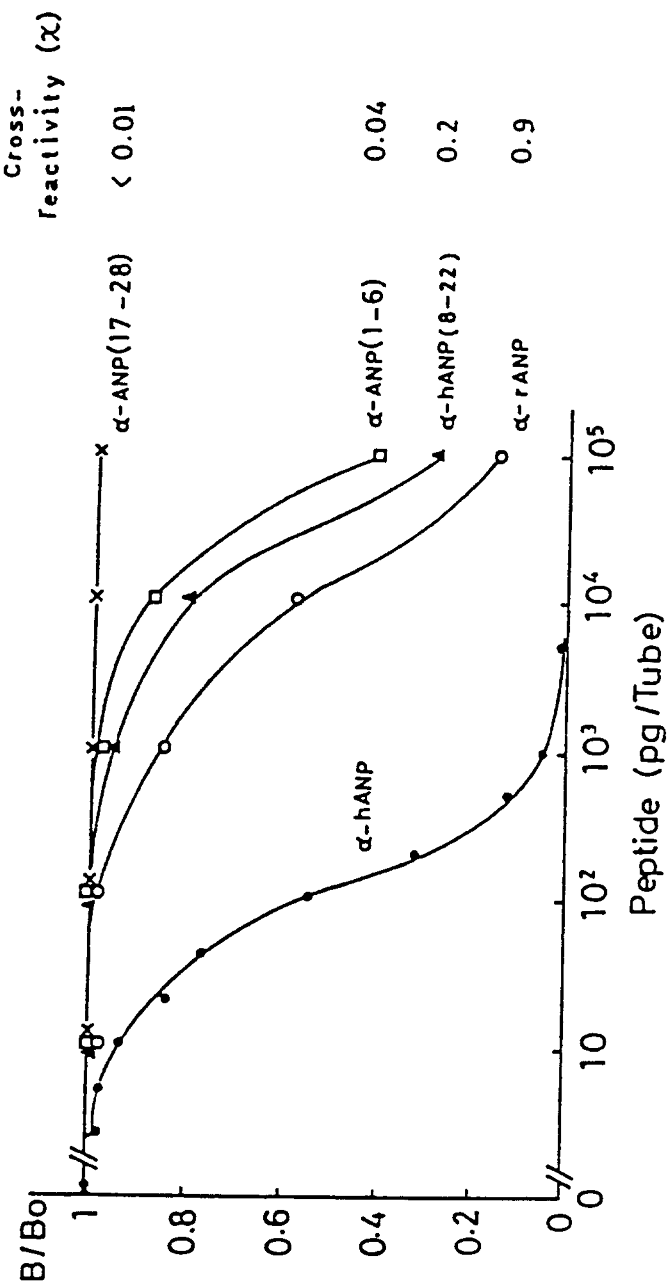
B/Bo
1
0.8
0.6
0.4
0.2
0
0
10
$10^2$
$10^3$
$10^4$
$10^5$
Peptide (pg/Tube)
α-hANP
α-ANP(17-28)
α-ANP(1-6)
α-hANP(8-22)
α-rANP
Cross-reactivity (%)
< 0.01
0.04
0.2
0.9

FIG. 2.

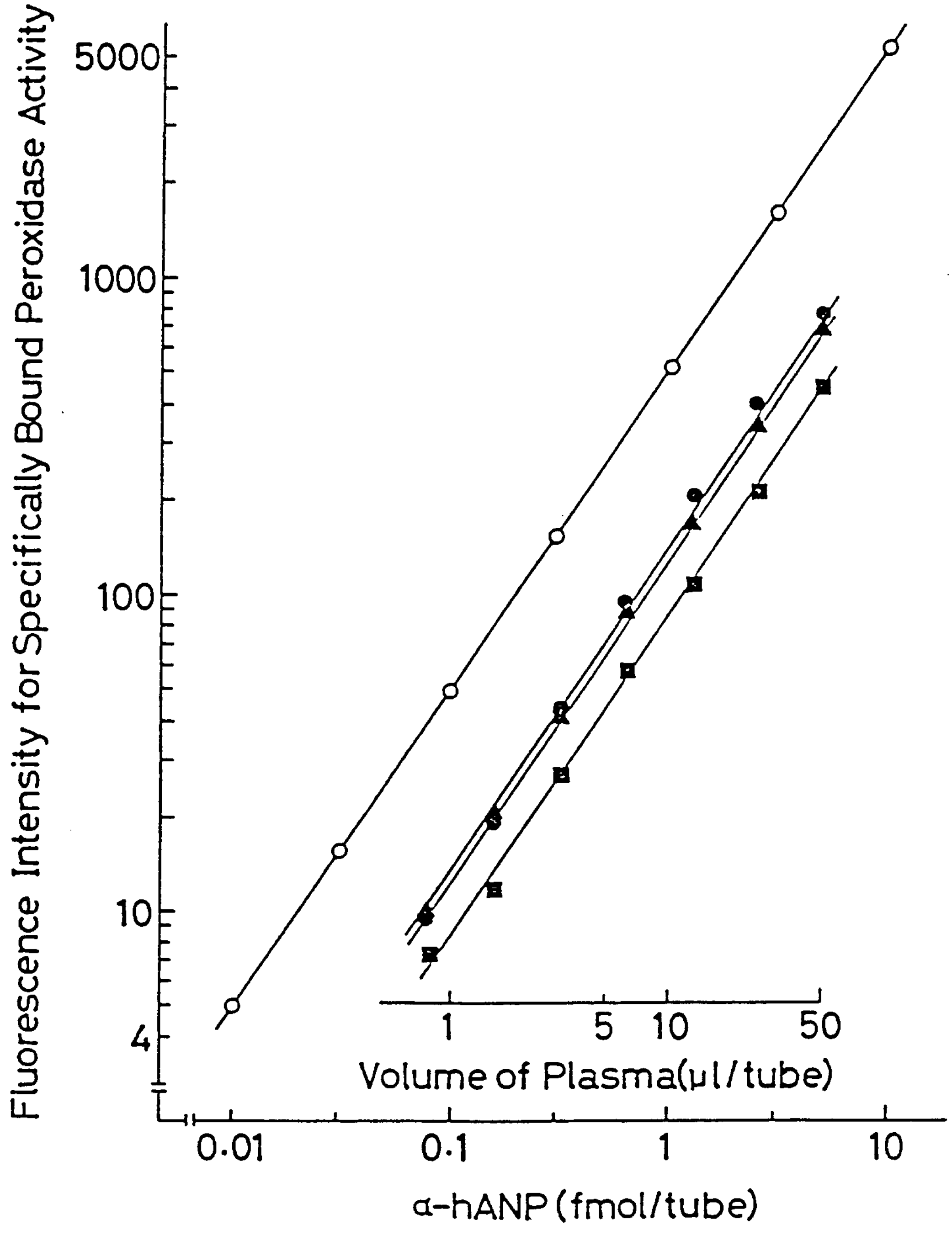
Fluorescence Intensity for Specifically Bound Peroxidase Activity
5000
1000
100
10
4
1
5
10
50
Volume of Plasma(μl/tube)
0.01
0.1
1
10
α-hANP (fmol/tube)

FIG. 3.

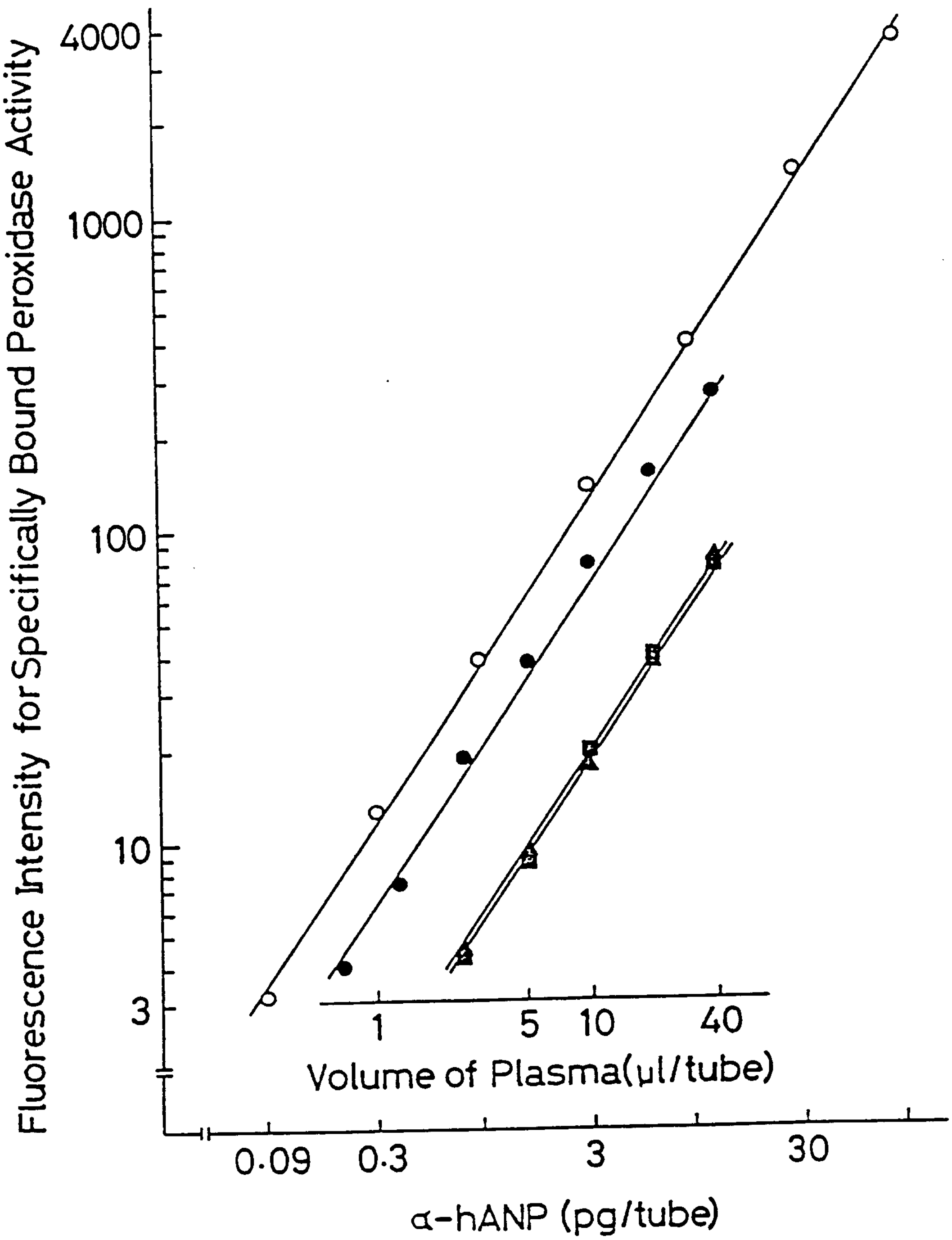
Fluorescence Intensity for Specifically Bound Peroxidase Activity
4000
1000
100
10
3
1
5
10
40
Volume of Plasma(μl/tube)
0·09
0·3
3
30
α-hANP (pg/tube)